(19) Europäisches Patentamt
European Patent Office
Office européen des brevets

(11) Publication number: **0 325 187 B1**

(12) # EUROPEAN PATENT SPECIFICATION

(45) Date of publication of patent specification: **11.11.92**

(51) Int. Cl.5: **C07D 211/90**, C07D 417/04, A61K 31/445

(21) Application number: **89100656.1**

(22) Date of filing: **16.01.89**

(54) **4-alkyl-1, 4-dihydropyridines with PAF-antagonist activity.**

(30) Priority: **21.01.88 DE 3801717**

(43) Date of publication of application:
**26.07.89 Bulletin 89/30**

(45) Publication of the grant of the patent:
**11.11.92 Bulletin 92/46**

(84) Designated Contracting States:
**AT BE CH ES FR GB GR IT LI LU NL SE**

(56) References cited:
**EP-A- 0 001 769**

**CHEMICAL ABSTRACTS, vol. 96, 1982, page 653, abstract no. 199478x, Columbus, Ohio, US; B. VIGANTE et al.: "1,4-Dihydropyridine-3 and 3,5-carbothiolic acid esters"**

**Thromb. Res., 31, 833-844 (1983)**

**Thromb. Res., 45, 427-435 (1987)**

**Adv. Inflamm. Res., 12, 135-157 (1988)**

**J. Med. Chem., 33, 3205-3210 (1990)**

(73) Proprietor: **ALTER, S.A.**
**Mateo Inurria, 30**
**E-28036 Madrid(ES)**

(72) Inventor: **Sunkel, Carlos**
**Almarza, 47**
**E-28033 Madrid(ES)**
Inventor: **Fau de Casa-Juana, Miguel**
**Torquemada, 17 - 4 2**
**E-28043 Madrid(ES)**
Inventor: **Santos, Luis**
**Conde de Penalver, 32 - 1 D**
**E-28006 Madrid(ES)**
Inventor: **Ortega, Pilar**
**Avda. Pio XII, 97-bis 4 C**
**E-28036 Madrid(ES)**
Inventor: **Priego, Jaime**
**Avda. Moratalaz, 66 - 3 B**
**E-28039 Madrid(ES)**
Inventor: **Sanchez-Crespo, Mariano**
**Rafael Herrera, 3 - 4 B**
**E-28036 Madrid(ES)**

(74) Representative: **Ungria Lopez, Javier et al**
**Avda. Ramon y Cajal, 78**
**E-28043 Madrid(ES)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 325 187 B1

## Description

The invention refers to a series of pharmaceutical compounds containing 1,4-dihydropyridines with an alkyl group in the 4-position of the ring, which have antagonist activity of the Platelet Activator Factor (PAF-aceter) and the methods of obtainment thereof.

The chemical structure of the PAF-aceter was identified as 1-0-hexadecyl/octadecyl-2-acetyl-sn-glycero-3-phosphocholine. This compound is a phospholipidc autokoide and an extremely powerful mediator of inflammatory reactions which is given off by various types of cells human tissues and laboratory animals. These cells are mainly granulocytes, basophils, eosinophils and neutrophils, tissue macrophages and monocytes of the peripheral blood, platelets, glandular epithelial cells, endothelial cells and neuronic tissue. The PAF-aceter is a powerful inducer of platelet aggregation and secretion and a powerful hypotensor of the systemic circulation. This effect is due to its capacity to promote peripheral vasodilatation, but its actions also have an influence on lung and heart circulation, since it produces a reduction of the myocardial contractility and reduction of the coronary flow. Another effect of the PAF-aceter is that it induces bronchial constriction at a dosis 100 times lwer than histamine.

Finally, the pro-inflammatory action thereof should be pointed out due to the capacity to stimulate the adhesion and aggregation of neutrophils followed by the release of lysosomal enzymes and activation of the cascade of arachidonic acid. In studies carried out on laboratory animals it has been demonstrated that PAF-aceter has an effect on vascular endothelium, promoting exudation of protein rich plasma and the adhesion of leukocytes. These data have been confirmed by experiments in which PAF-aceter was injected in the skin of healthy volunteers.

It may cause disorders of heart rate and the above mentioned reduction of contractile force of the heart. Concerning liver tissue it stimulates glycogenolysis at concentrations 10,000 times lower than those required of epinephrine and glucagon. More recently, actions on the central nervous system, on the physiology of reproduction and on immunoregulation have been described.

These facts, along with the demonstration of the existence of PAF-aceter in biological fluids in different experimental and clinical situations and the results obtained with the use of pharmacological antagonists in experimental models and in clinical studies, suggest a pathogenic role of PAF-aceter in certain human diseases.

On this basis, the specific inhibitors of biosynthesis and/or of the effects of PAF-aceter could represent a new type of therapeutic agents, especially in lung diseases such as bronchial asma, allegic pneumonitis and respiratory distress in adults, in which some PAF antagonists have demonstrated favourable effects in the first clinical tests carried out. Likewise, a series of PAF-aceter antagonists have reduced or put an end to reactions of anaphylaxis, hypersensitivity, endotoxic shock and gastric ulcerations in experimental studies. The participation of PAF-aceter in an entire series of pathological states based on immunoallergy such as inflammatory processes of the skin, psoriasis, glomerulonephritis and rejection of transplants.

Some compounds with antagonist activity of the PAF-aceter receptor which may be grouped into four basic types:

- Antagonists based on modifications of the chemical structure of PAF-aceter (structural analogues)
- Natural products and derivatives thereof
- Synthetic structures
- Already existing drugs and derivatives thereof, for example, certain benzodiazepins, allergy drugs, anti-inflammatories, $\alpha$-adrenergic drugs.

Some antagonists of $Ca^{++}$ also have PAF antagonist properties, such as the case of Diltiazem and Gallopamil, however, not all $Ca^{++}$ channel blocking agents have this activity. Hence, 1,4-dihydropyridines, such as Nifedipin, only demonstrate a very weak activity, since the concentrations required to obtain an effect are 1,000,ooo times higher than those that inhibit calcium flows in other cel,ls and, at least, 1,000 times higher than those of specific PAF-aceter antagonists.

Surprisingly, some 1,4-dihydropyridines which have an aliphatic group in the 4-position of the ring show a strong PAF-antagonist activity not described until now.

The object of the invention is, therefore, pharmaceutical compositions with PAF-antagonist activity which contain as active substances one or more compounds of general formula I

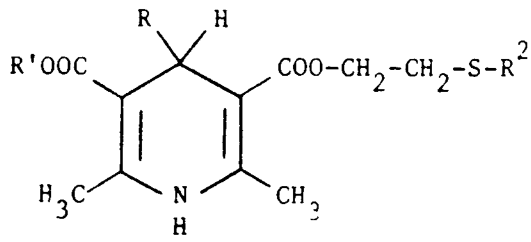

I

wherein

- R is a $C_1$-$C_4$ saturated straight or branched chain alkyl group
- R′ may be a C--$C_6$ saturated straight or branched chain alkyl group, which may be interrupted by an oxygen atom, it may also represent the 2-tetrahydrofurfuryl group,
- $R^2$ may be a $C_1$-$C_4$ saturated straight chain alkyl group or may represent a phenyl group

One of the compounds included in general formula I, when R = $CH_3$, R′ = $CH_3$-$CH_2$- and $R^2$ = phenyl, is described in the bibliography (KHIM, GETEROTSIKL, SOEDIN., 219 (1982.))

The pharmacological activity of the compounds of formula I has been established as indicated hereafter.

The compounds can be obtained in accordance with the methods already known in literature.

Hence,

a) A compound of formula II

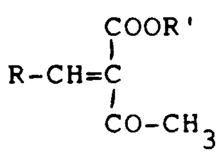

II

wherein R and R′ are as defined above, is reacted with a compound of formula III

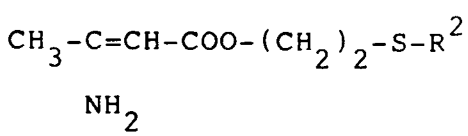

III

wherein $R^2$ is as defined above, to give a compound of formula I; or

b) A compound of formula IV

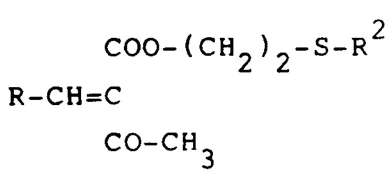

IV

wherein R and $R^2$ are defined as above, is reacted with a compound of formula V

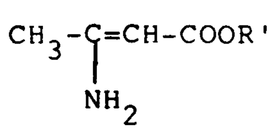

V

wherein R′ is as defined above, to give a compound of formula I; or
c) A compound of formula VI

$CH_3$-CO-$CH_2$-COOR′ VI

wherein R′ is as defined above, is reacted with a compound of formula III, wherein $R^2$ is as defined above, and a compound of formula VII

R-CHO VII

wherein R is as defined above to give a compound of formula I; or
d) A compound of formula VIII

$CH_3$-CO-$CH_2$-COO-$(CH_2)_2$-S-$R^2$ VIII

wherein $R^2$is as defined above, is reacted with a compound of formula V, wherein R′ is as defined above, and a compound of formula VII, wherein R is defined as above, to give a compound of formula I; or
e) A compound of formula VIII, wherein $R^2$ is defined as above, is reacted with a compound of formula VI, , wherein R′ is defined as above, and a compound of formula VII, wherein R is defined as above, in the presence of ammonia, to give a compound of formula I.

The invention also refers to those embodiments of the process according to which one starts from a compound obtainable as an intermediate in any step of the process and the remaining steps of the process are carried out, or the process is interrupted in any step, or in which a starting product is formed under the reaction conditions or in which a reaction compound is present in the form of salts thereof.

The mixtures of diastereoisomers or enantiomers obtained can be separated thanks to the different physical-chemical properties of the components, by means of known methods, such as, for example, fractionated recrystallization and/or chromatography, by assymetric induction reactions or by means of the use of microorganisms.

The starting compounds are known, or in the event they are new, they can be obtained by known methods.

The compounds of formula I can be used as medication to be administered orally, rectally, topically, parenterally or inhalation, in the form of a pharmaceutical preparation which contains at least one of the compounds of formula I in combination with a pharmaceutically acceptable excipient. The pharmaceutical preparations may come, for example, in the form of tablets, sugar-coated pills, capsules, suppositories, syrups or aerosols. Of course, the amount of active compound is between 0.1 and 99 % by weight of the preparation, preferably between 2 and 50 % by weight in oral preparations. The daily dose of the active substance depends on the type of administration and, in general, between 25 and 100 mg. are administered orally, between o.1 and 50 mg. per dose are administered intravenously or intramuscularly and solutions containing between 0.1 and 0.5 % of the active product are used for inhalattions.

Example 1

2-(ethylthio)ethyl-2,6-dimethyl-4-methyl-5-methoxycarbonyl-1,4-dihydropyridin-3-carboxylate

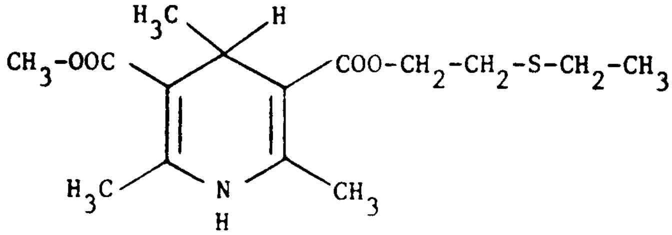

A mixture made up of 28.54 g (0.15 moles) of 2-(ethylthio)ethyl-ethylacetate, 17.27 g (0.15 moles) of methyl 3-aminocrotonate and 8.5 ml (6.61 g; 0.15 moles) of acetaldehyde in 120 ml. of ethanol, is heated to reflux with stirring during 8 hours. After cooling the resulting solution to -10 ° C, a light yellow solid is obtained ; melting point: 84-6 ° C (recrystallized in ethanol.) The yield of the reaction is around 49 %.

| Analysis for $C_{15}H_{23}NO_4S$ | | | | |
|---|---|---|---|---|
| | % C | % H | % N | % S |
| Calculated | 57.48 | 7.40 | 4.47 | 10.23 |
| Found | 57.37 | 7.61 | 4.60 | 10.34 |

I.R. spectrum (KBr) ν ($cm^{-1}$):
3340, 3240, 2960, 1700, 1650 1490, 1430, 1350, 1290, 1220, 1200, 1140, 1090, 1050, 770, 700.
NMR spectrum ( δ , $CDCl_3$) p.p.m. :
(1H, sa); 4.3 (2H, t); 3.9 to 3.7 (1H+3H, m+s); 2.9 to 2.4 (2H+ 2H, t+c); 2.3 (6H, s); 1.3 (3H, t); 1 (3H, d.)

Example 2

2-(methylthio)ethyl-2,6-dimethyl-4-methyl-5-methoxycarbonyl-1,4-dihydropyridin-3-carboxylate

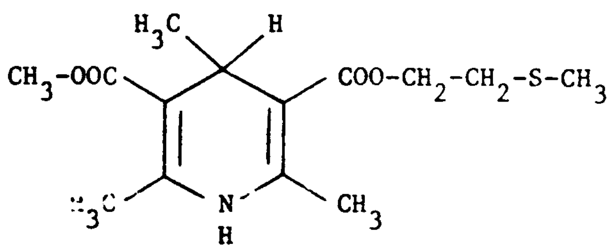

A mixture made up of 20 g (0.11 moles) of 2-(methylthio) ethyl-acetylacetate, 13.07 g (0.11 moles) of methyl 3-aminocrotonate and 6.42 ml (0.11 moles) of acetaldehyde in 110 ml of ethanol, is heated to reflux with stirring for 8 hours. After evaporation of 50 ml. of the previous solvent and cooling the resulting solution to -10° C, a white solid is obtained; melting point; 92-5° (recrystallization in ethanol.) The yield of the reaction is 47%.

| Analysis for $C_{14}H_{21}NO_4S$ | | | | |
|---|---|---|---|---|
| | % C | % H | % N | % S |
| Calculated | 56.17 | 7.07 | 4.68 | 10.71 |
| Found | 55.85 | 7.36 | 4.76 | 11.03 |

I.R. spectrum (KBr) ν ($cm^{-1}$):
3340, 2950, 2920, 1700, 1650, 1490, 1430, 1350, 1290, 1220, 1200, 1130, 1090, 1050, 980, 770, 700.
NMR spectrum (δ, $CDCl_3$) p.p.m. :
6.4 (1H, sa); 4.3 (2H, t); 3.8 to 3.6 (1H+3H, m+s); 2.7 (2H, t); 2.3 (6H, s); 2.1 (3H, s); 1 (3H, d.)

Example 3

2,6-(methylthio)ethyl-2,6 dimethyl-5-ethoxycarbonyl-4-methyl-1,4-dihydropyridin-3-carboxylate

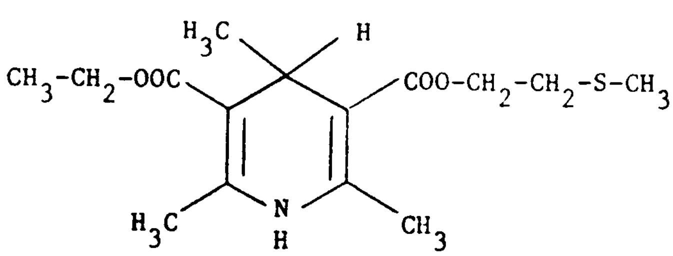

A mixture made up of 20 g (0.11 moles) of 2-(methylthio) ethyl-acetylacetate, 14.66 g (0.11 moles) of ethyl 3-aminocrotonate and 6.41 ml (5 g; 0.11 moles) of acetaldehyde in 110 ml. of ethanol, is heated to reflux with stirring for 8 hours . After evaporation of 50 ml. of the previous solvent and cooling the resulting solution to -10° C, a light yellow solid is obtained; melting point: 87-90° C (recrystallized in aqueous ethanol 70:30.) The yield of the reaction is 82 %.

| Analysis for $C_{15}H_{23}NO_4S$ | | | | |
|---|---|---|---|---|
| | % C | % H | % N | % S |
| Calculated | 57.48 | 7.40 | 4.47 | 10.23 |
| Found | 57.69 | 7.48 | 4.48 | 10.37 |

I.R. spectrum (KBr) ν ($cm^{-1}$):
3340, 2950. 2920, 1700, 1650, 1490, 1370, 1300, 1210, 1130, 1090, 1050, 980, 770, 690.
NMR spectrum ( δ ,$CDCl_3$) p.p.m. :
6.6 (1H, sa); 4.4 to 3.7 (5H, m); 2.8 (2H, t); 2.3 (6H, s); 2.2 (3H, s); 1.3 (3H, t); 1 (3H, d.)

Example 4

2-(ethylthio)ethyl-2,6-dimethyl-5-ethoxycarbonyl-4-methyl-1,4-dihydropyridin-3-carboxylate

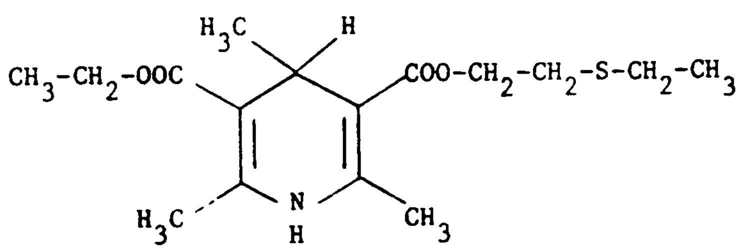

A mixture made up of 20 g (0.11 moles) of 2-(ethylthio) ethyl-acetylacetate, 13.58 g (0.11 moles) ethyl-3-aminocrotonate and 5.94 ml (4.63 g; 0.11 moles) of acetaldehyde in 100 ml of ethanol is heated to reflux with stirring for 8 hours. After evaporation of 50 ml. of the solvent and cooling the resulting solution to -10° C, a white solid is obtained; melting point: 83-5° C (recrystallized in ethyl acetate.) The yield of the reaction is 53 %.

| Analysis for $C_{16}H_{25}NO_4S$ | | | | |
|---|---|---|---|---|
| | % C | % H | % N | % S |
| Calculated | 58.69 | 7.70 | 4.28 | 9.79 |
| Found | 58.51 | 7.98 | 4.66 | 10.24 |

I.R. spectrum (KBr) ν ($cm^{-1}$):
3380, 2980, 1710, 1650, 1490, 1380, 1300, 1220, 1140, 1100, 1060, 990, 780, 700.
NMR spectrum ( δ, $CDCl_3$) p.p.m. :
6.5 (1H, sa); 4.4 to 3.7 (4H + 4H, td + m); 2.6 (2H + 2H, t + t); 2.2 (6H, s); 1.3 (6H, t); 1 (3H, d.)

Example 5

2-(methylthio)ethyl-2,6-dimethyl-4-methyl-5-(2-methoxyethoxycarbonyl)-1,4-dihydropyridin-3-carboxylate

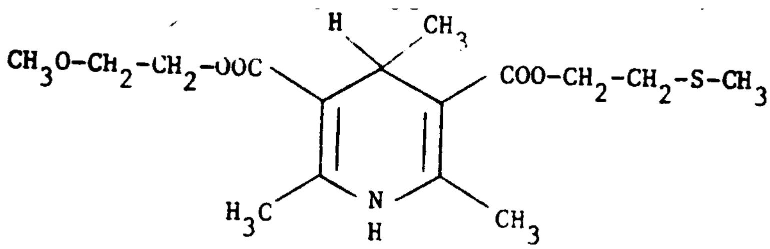

A mixture made up of 10.5 g (0.06 moles) of 2-(methylthio)ethyl-3-aminocrotonate and 11.16 g (0.06 moles) of 2 - methoxyethyl-α-ethylidenacetylacetate in 60 ml. of ethanol is heated to reflux with stirring for 12 hours. After evaporation of the solvent at reduced pressure, dissolution of the residue in 20 ml. of ethyl acetate to boiling and cooling of the resulting solution to 5° C, a solid is obtained; melting point: 58-60° C (recrystallized in ethanol.) The yield of the reaction is 51 %.

| Analysis for $C_{16}H_{25}NO_5S$ | | | | |
|---|---|---|---|---|
| | % C | % H | % N | % S |
| Calculated | 55.96 | 7.34 | 4.08 | 9.34 |
| Found | 56.16 | 7.65 | 3.98 | 9.68 |

I.R. spectrum (KBr) ν ($cm^{-1}$):
3360, 2950, 1700, 1640, 1480, 1380, 1290, 1210, 1130, 1050, 980, 770, 690
NMR spectrum (δ,DMSO-$D_6$) p.p.m. :
8.5 (1H, sa); 4.2 (4H, td); 3.8 to 3.4 (3H, m); 3.3 (3H, s); 2.7 (2H, t); 2.2 (6H, s); 2.1 (3H, s); 0.9 (3h, d.)

Example 6

2-(methylthio)ethyl-2,6-dimethyl-4-ethyl-5-ethoxycarbonyl-1,4-dihydropyridin-3-carboxylate

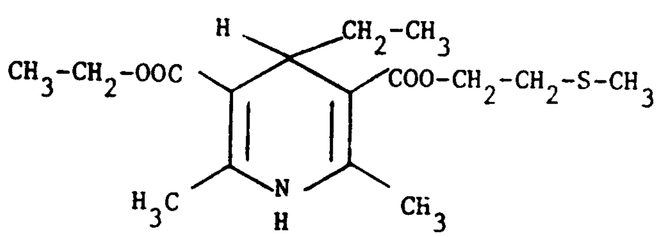

A mixture made up of 15 g (0.09 moles) of 2-(methylthio) ethyl-acetalacetate, 10.99 g (0.09 moles) of ethyl-3-aminocrotonate and 6.5 ml (4.94 g; 0.09 moles) of propionaldehyde in 85 ml. of ethanol, is heated to reflux with stirring for 10 hours. After evaporation of the solvent at reduced pressure, the residue is dissolved in 10 ml. of ethyl acetate to boiling and the resulting solution is cooled to -10° C. In this way a white solid is obtained: melting point: 96-8° C (recrystallized in DMF-$H_2O$.) The yield of the reaction is 65 %.

| Analysis for $C_{16}H_{25}NO_4S$ | | | | |
|---|---|---|---|---|
| | % C | % H | % N | % S |
| Calculated | 58.69 | 7.70 | 4.28 | 9.79 |
| Found | 58.94 | 8.00 | 4.21 | 9.80 |

I.R. spectrum (KBr) ν ($cm^{-1}$):
3360, 3240, 2980, 2940, 2880, 1700, 1660, 1490, 1450, 1380, 1220, 1140, 1080, 1010, 890, 790, 770, 730.
NMR spectrum ( δ , $CDCl_3$) p.p.m. :

6.9 (1H, s); 4.4 to 3.8 (5H, m); 2.7 (2H, t); 2.3 (6H, s); 2.2 (3H, s); 1.3 (5H, td); 0.8 (3H,t.)

Example 7

2-(methylthio)ethyl-2,6-dimethyl-5-ethoxycarbonyl-4-n-propyl-1,4-dihydropyridin-3-carboxylate

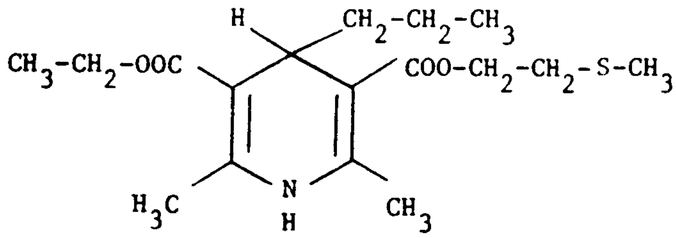

A mixture made up of 15 g (0.09 moles) of 2-(methylthio)ethyl-acetylacetate, 10.99 g (0.09 moles) of ethyl-3-aminocrotonate and 7.7 ml (6.14 g; (0.09 moles) of butyraldehyde in 85 ml. of absolute ethanol is heated to reflux with stirring for 10 hours. After evaporation of 45 ml ofsolvent at reduced pressure, the resulting solution is cooled to -10° C. In this way a pale yellow crystalline solid is obtained; melting point: 94-96° C (recrystallized in ethanol.) The yield of the reaction is 56 %.

| Analysis for $C_{17}H_{27}NO_4S$ | | | | |
|---|---|---|---|---|
| | % C | % H | % N | % S |
| Calculated | 59.80 | 7.97 | 4.10 | 9.39 |
| Found | 59.64 | 8.02 | 3.96 | 9.35 |

I.R. spectrum (KBr) $\nu$ ($cm^{-1}$):
3340, 3250, 2960, 2920, 1710, 1660, 1500, 1380, 1300, 1220, 1150, 1090, 1010, 800, 740, 680.
NMR spectrum ($\delta$, $CDCl_3$) p.p.m. :
6.5 (1H, sa); 4.4 to 3.8 (4H+1H,m); 2.7 (2H, t); 2.3 (6H, s); 2.1 (3H, s); 1.4 to 0.6 (10H, t+m.)

Example 8

2-(ethylthio)ethyl-2,6-dimethyl-5-ethoxycarbonyl-4-n-propyl-1,4-dihydropyridin-3-carboxylate

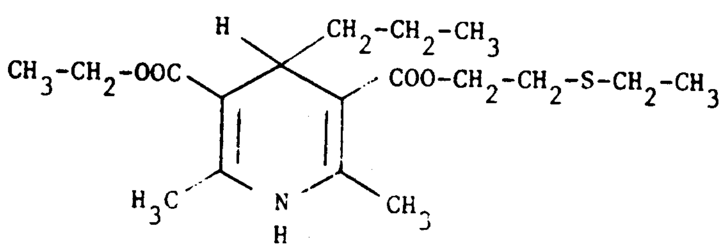

A mixture made up of 15 g (0.08 moles) of 2-(ethylthio) ethyl acetylacetate, 10.18 g (0.08 moles) of ethyl-acetylacetate, 10.18 g (0.08 moles) of ethyl-3-aminocrotonate and 7.2 ml (5.69 g; 0.08 moles) of butyraldehyde in 80 ml of absolute ethanol, is heated to reflux with stirring for 10 hours. After evaporation of 40 ml of the solvent at reduced pressure and cooling of the resulting solution to -10° C, a crystalline yellow solid is prisms is obtained; melting point: 68-70° C (recrystallized in ethanol.) The yield of the reaction is 62 %.

| Analysis for $C_{18}H_{29}NO_4S$ | | | | |
|---|---|---|---|---|
| | % C | % H | % N | % S |
| Calculated<br>Found | 60.82<br>60.92 | 8.22<br>8.44 | 3.94<br>4.03 | 9.02<br>9.25 |

IR spectrum (KBr) ν ($cm^{-1}$):
3370, 2980, 2940, 1720, 1660, 1500, 1300, 1220, 1150, 1090 1020, 800, 790, 740
NMR spectrum (δ, $CDCl_3$) p.p.m. :
6.5 (1H, sa); 4.4 to 3.8 (4H + 1H, m); 2.9 to 2.5 (2H + 2H, 2t); 2.3 (6 H, s); 1.4 to 0.7 (13H, t + m.)

Example 9

2-(phenylthio)ethyl-2,6-dimethyl-5-ethoxycarbonyl-4-methyl-1,4-dihydropyridin-3-carboxylate

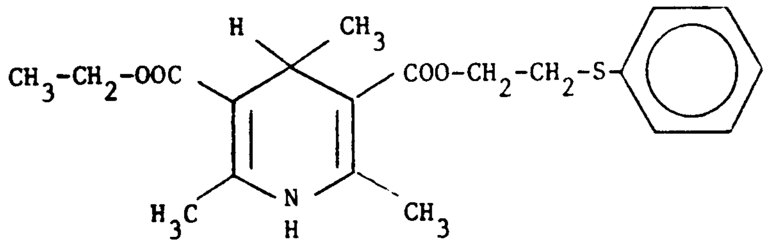

(A) 2-(phenylthio)ethyl-acetylacetate

28.5 ml (31.24 g, 0.37 moles) of diketene are added drop by drop and with stirring to a mixture made up of 50 ml (57.30 g; 0.37 moles) of 2-hydroxyethylphenylsulfide and 0.4 ml triethylamine previously heated to 80° C. The rate of addition is adjusted so that the temperature is kept between 85-90° C. Once the adding has ended the reaction mixture is kept at 90° C for 3 hours with stirring. The desired product is isolated by reduced pressure distillation of the previous mixture, resulting in a colorless liquid; melting point: 143-5° C/0.5 Torr. The yield of the reaction is 86 %.
I.R. spectrum (NaCl) ν ($cm^{-1}$):
3060, 2950, 1750, 1720, 1650, 1580, 1480, 1440, 1360, 1320, 1150, 1020, 740, 690.
NMR spectrum (δ, $CDCl_3$) p.p.m. :
7.2 (5H m); 4.2 (2H t); 3.3 (2H, s); 3.1 (2H, t); 2.1 (3H, s.)

(B) 2-(phenylthio)ethyl-2,6-dimethyl-5-ethoxycarbonyl-4-methyl-1,4-dihydropyridin-3-carboxylate

A mixture made up of 10 g (0.04 moles) of 2-(phenylthio) ethyl-acetylacetate, 5.42 g (0.04 moles) of ethyl-3-aminocrotonate and 2.4 ml (1.85 g; 0.04 moles) of acetaldehyde in 45 ml of ethanol, is heated to reflux with stirring for 10 hours. After evaporation of 25 ml of the solvent at reduced pressure and cooling of the resulting solution to -10° C, a crystalline yellow solid is obtained: melting point: 80-2° C (recrystallized in ethanol.) The yield of the reaction is 49%.

| Analysis for $C_{20}H_{25}NO_4S$ | | | | |
|---|---|---|---|---|
| | % C | % H | % N | % S |
| Calculated<br>Found | 63.98<br>64.15 | 6.71<br>7.01 | 3.73<br>3.77 | 8.54<br>8.27 |

IR spectrum (KBr) ν ($cm^{-1}$):
3340, 2950, 1690, 1640, 1480, 1380, 1290, 1210, 1130, 1050, 770, 720, 680.
NMR spectrum (δ, $CDCl_3$) p.p.m. :

7.2 (5H, m); 6.8 (1H, sa); 4.4 to 3.8 (4H+H, m); 3.2 (2H, t); 2.2 (6H. s); 1.3 (3H. t); 1.0 (3H, d.)

Example 10

2-(phenylthio)ethyl-2,6-dimethyl-4-methyl 5-(2-tetrahydrofurfuryloxycarbonyl)-1,4-dihydropyridin-3-carboxylate

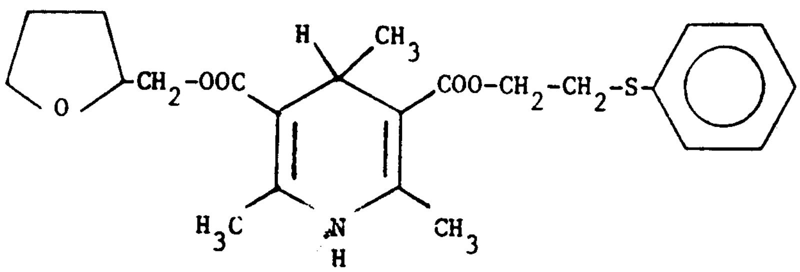

A mixture made up of 15 g (0.06 moles) of 2-(phenylthio) ethyl-acetylacetate (obtained as indicated in example 9,) 3.6 ml (2.77 g; 0.06 moles) of acetaldehyde and 11.66 g (0.06 moles) of 2-tetrahydrofurfuryl-3-aminocrotonate in 60 ml. of absolute ethanol is heated to reflux with stirring for 12 hours. At the end of said time, the resulting solution is purified by passing through activated carbon-infusoria earth (1:1) and then the solvent is removed at reduced pressure. In this way the product is obtained in the form of a yellow oil. The yield of the reaction is 65 %.
I.R. spectrum (NaCl) ν ($cm^{-1}$):
3360, 3100, 2980, 2880, 1700, 1670, 1630, 1500, 1450, 1390, 1310, 1280, 1230, 1150, 1110, 1060, 1000, 780, 750, 700.
NMR spectrum (δ, $CDCl_3$) p.p.m. :
7.2 (5H, m); 5.7 (1H, sa); 4.4 to 3.6 (8H, m); 3.2 (2H, t); 2.2 (6H, s); 2 to 1.7 (4H, m); 1.0 (3H, d.)

Example 11

2-(phenylthio)ethyl-2,6-dimethyl-5-isopropoxycarbonyl-4-methyl-1,4-dihydropyridin-3-carboxylate

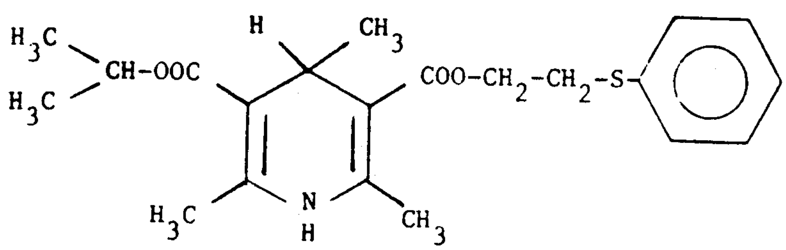

A mixture made up of 15 g (0.06 moles of 2-(phenylthio) ethyl-acetylacetate (obtained as indicated in example 9,) 9.01 g (0.06 moles) of isopropyl 3-aminocrotonate and 3.6 ml (2.77 g; 0.06 moles) of acetaldehyde in 60 ml. of absolute ethanol, is heated to reflux with stirring for 12 hours. At the end of said time, the resulting solution is purified by passing through activated carbon-infusoria earth (1:1) and finally the solvent is removed at reduced pressure. In this way the product is obtained in the form of a pale yellow oil. The yield of the reaction is 67 %.
I.R. spectrum (NaCl) ν ($cm^{-1}$):
3350, 3100, 2980, 1700, 1670, 1500, 1450, 1390, 1300, 1280, 1230, 1150, 1110, 1060, 780, 740, 700
NMR spectrum (δ, $CDCl_3$) p.p.m. :
7.2 (5H, m); 6.0 (1H, sa); 5.0 (1H, h); 4.3 (2H, t); 3.8 (1H, c); 3.2 (2H, t); 2.2 (6H, s); 1.3 (6H, d); 1.0 (3H, d.)

Example 12

2-(phenylthio)ethyl-2,6-dimethyl 4-methyl-5-methoxycarbonyl-1,4-dihydropyridin-3-carboxylate

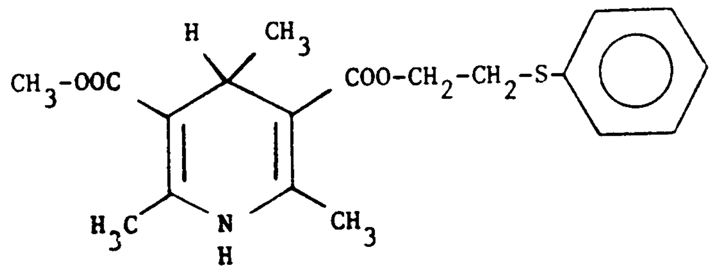

A mixture nade up of 15 g (0.06 moles) of 2-(phenylthio) ethyl-acetylacetate (obtained as indicated in example 9,) 3.6 ml (2.77 g; 0.06 moles) of acetaldehyde and 7.25 g (0.06 moles) of methyl 3-aminocrotonate in 60 ml of absolute ethanol is heated to reflux with stirring for 12 hours. At the end of said time, the solvent is evaporated at reduced pressure and the resulting oil is subjected to purification by column chromatography (adsorbent: Silicagel 60, "Merck;" Eluent: Toluene/Acetone (9:1).) In this way a white solid is obtained; melting point: 69-71.5° C. The yield of the reaction is 59 %.
I.R. spectrum (KBr) ($cm^{-1}$):
3350, 2950, 1700, 1650, 1500, 1440, 1300, 1220, 1140, 1100, 1060, 780, 740, 700, 690.
NMR spectrum ( , $CDCl_3$) p.p.m. :
7.2 (5H, m); 6.3 (1H, sa); 4.3 (2H, t); 3.9 to 3.6 (1H+3H, m+s); 3.2 (2H, t); 2.2 (6H, s); 1.0 (3H, d.)

PHARMACOLOGICAL STUDIES

1. STUDIES ON WASHED PLATELETS

1.1.Aggregation

The suspensions of washed platelets were obtained from the blood of rabbits (male, albino, New Zealand) anticoagulated with a citric acid-dextrose solution in the proportion 1:6 (v:v.)

The platelet rich plasma (PRP) was obtained by centrifuging the blood samples at 100xg for 10 minutes and the platelet suspensions by centrifuging the PRP at 1,800xg for 15 minutes at 4° C. The pellet thus obtained was washed twice with Tyrode buffer containing citric acid, $PGE_1$ and apirase, pH 6.5. The platelets thus washed were finally resuspended in Hepes-Tyrode buffer, pH 7.35, supplemented with bovine albumin serum at 0.35 %; $Ca^{++}$ 2 mM.

The final concentration of platelets was adjusted to 300,000 platelets/µl. The aggregation was measured turbidimetrically using a lumiaggregometer (Chrono-Log Co., Haventon, PA, USA) at 37° C under stirring at 1,100 r.p.m. The study was also carried out on aliquots of PRP pre-incubated with the compounds under study at 37° C for 5 minutes. The concentration of the antagonists were in all cases 10 µM and aggregation was induced by adding L-PAF diluted in 5 µl of Hepes buffer/albumin, with a final concentration 1.9 x $10^{-9}$M.

1.2. Release reaction

The platelet secretion was measured in the presence of luciferin/luciferase with ATP release in accord with the method described by Feinman et al. (1977.)

2. STUDIES ON ANESTHETIZED RATS

Hypotension induced by an intravenous injection of PAF-aceter.

Male Sprague-Dawley rats anesthetized with pentobarbital (50 mg/kg., intraperitoneally) were used. 5 mg/kg of the compounds under study were administered intravenously three minutes after injecting PAF-aceter (0.66 µg/kg.) The changes in the average blood pressure were observed for 30 minutes.

Table 1

| EFFECT ON AGGREGATION AND RELEASE REACTION INDUCED BY 1-PAF ON RABBIT PLATELETS | | |
|---|---|---|
| COMPOUND ACCORDING TO EXAMPLE | % INHIBITION | |
| | AGGREGATION | RELEASE REACTION |
| 1 | 44 | 96 |
| 2 | 26 | 75 |
| 3 | 21 | 78 |
| 4 | 32 | 89 |
| 5 | -- | -- |
| 6 | 32 | 89 |
| 7 | 14 | 50 |
| 8 | 30 | 69 |
| 9 | 100 | 100 |
| 10 | 11 | 49 |
| 11 | 33 | 89 |
| 12 | 100 | 100 |
| The data are the average of six experiments carried out in triplicate. Concentration of 1-PAF = 1.92 nM. The concentration of the compounds was in all cases 10 µM. Pre-incubation time: 5 minutes | | |

| INHIBITORY EFFECT ON HYPOTENSION INDUCED BY 1-PAF ON ANESTHETIZED RATS | | |
|---|---|---|
| COMPOUND ACCORDING TO EXAMPLE | % INHIBITION OF HYPOTENSION | |
| | 3 min.* | 20 min.* |
| 1 | 9 | 0 |
| 2 | 14 | 51 |
| 3 | -19[a] | 0 |
| 4 | 5 | 33 |
| 5 | -- | -- |
| 6 | 21 | 30 |
| 7 | -31[a] | -82[a] |
| 8 | -38[a] | -115[a] |
| 9 | 32 | 30 |
| 10 | 8 | 36 |
| 11 | 0 | 18 |
| 12 | 61 | 57 |
| The data are the average of 5 experiments. The dose of compound administered intravenously was in all cases 5 mg/kg. | | |

(*) 1-PAF (0.66 µg/kg) was injected 3 and 20 minutes before the intravenous injection of the compounds
(a) The products increased hypotension induced by 1-PAF.

## Claims

**Claims for the following Contracting States : AT, BE, CH, FR, GB, IT, LI, LU, NL, SE**

**1.** 4-alkyl-1,4-dihydropyridines with PAF-antagonist activity of formula I

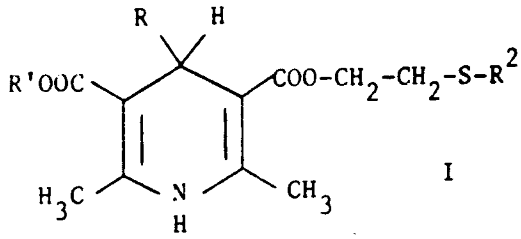

I

wherein

R is a $C_1$-$C_4$ saturated straight or branched chain alkyl group,

R′ may be a $C_1$-$C_6$ saturated straight or branched chain alkyl group, which may be interrupted by an oxygen atom. It may also represent the 2-tetrahydrofurfuryl group,

$R^2$ may be a $C_1$-$C_4$ saturated straight chain alkyl group or may represent a phenyl group, with the proviso that the compound wherein R is methyl, R′ is ethyl and $R^2$ is phenyl is excluded from said formula I.

**2.** A compound according to claim 1, selected from among the following:

a) 2-(ethylthio)ethyl-2,6-dimethyl-4-methyl-5-methoxycarbonyl-1,4-dihydropyridin-3-carboxylate,
b) 2-(methylthio)ethyl-2,6-dimethyl-4-methyl-5-methoxycarbonyl-1,4-dihydropyridin-3-carboxylate,
c) 2-(methylthio)ethyl-2,6-dimethyl-5-ethoxycarbonyl-4-methyl-1,4-dihydropyridin-3-carboxylate,
d) 2-(ethylthio)ethyl-2,6-dimethyl-5-ethoxycarbonyl-4-methyl-1,4-dihydropyridin-3-carboxylate,
e) 2-(methylthio)ethyl-2,6-dimethyl-4-methyl-5-(2-methoxyethoxycarbonyl)-1, 4-dihydropyridin-3-carboxylate.
f) 2-(methylthio)ethyl-2,6-dimethyl-4-ethyl-5-ethoxycarbonyl-1,4-dihydropyridin-3-carboxylate,
g) 2-(methylthio)ethyl-2,6 dimethyl-5-ethoxycarbonyl-4-n-propyl-1,4-dihydropyridin-3-carboxylate,
h) 2-(ethylthio)ethyl-2,6-dimethyl-5-ethoxycarbonyl-4-n-propyl-1,4-dihydropyridin-3-carboxylate,
i) 2-(phenylthio)ethyl-2,6-dimethyl-4-methyl-5-(2-tetrahydrofurfuryloxycarbonyl)-1,4-dihydropyridin-3-carboxylate,
j) 2-(phenylthio)ethyl-2,6-dimethyl-5-isopropoxycarbonyl-4-methyl-1,4-dihydropyridin-3-carboxylate
k) 2,6-(phenylthio)ethyl-2,6-dimethyl-4-methyl-5-methoxy-carbonyl-1,4-dihydropyridin-3-carboxylate.

**3.** Process for the preparation of 4-alkyl-1,4-dihydropyridines of formula (I):

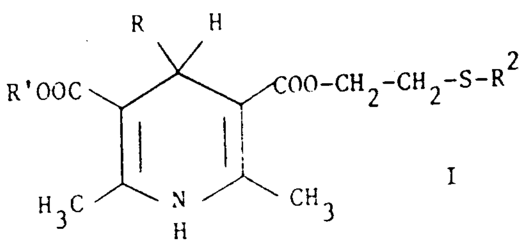

I

wherein,

R is a $C_1$-$C_4$ saturated straight or branched chain alkyl group,

R' may be a $C_1$-$C_6$ saturated straight or branched chain alkyl group, which may be interrupted by an oxygen atom. It may also represent the 2-tetrahydrofurfuryl group,

$r^2$ may be a $C_1$-$C_4$ saturated straight chain alkyl group or may represent a phenyl group,

with the proviso that the compound wherein R is methyl, R' is ethyl and $R^2$ is phenyl is excluded from said formula I

characterized in that:

a) A compound of formula II

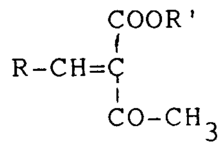

II

wherein R and R' are as defined above, is reacted with a compound of formula III

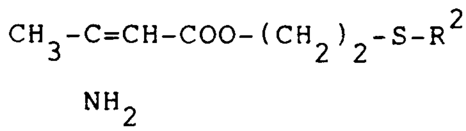

III

wherein $R^2$ is as defined above, to give a compound of formula I; or

b) A compound of formula IV

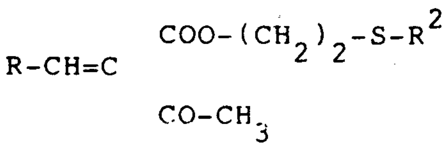

IV

wherein R and R' are as defined above, is reacted with a compound of formula V

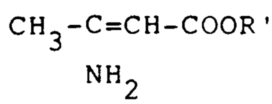

V

wherein R' is as defined above, to give a compound of formula I; or

c) A compound of formula VI

$CH_3$-CO-$CH_2$-COOR'    VI

wherein R' is as defined above, is reacted with a compound of formula III, wherein $R^2$ is as defined above, and a compound of formula VII

R-CHO    VII

wherein R is as defined above to give a compound of formula I; or

d) A compound of formula VIII

$CH_3$-CO-$CH_2$-COO-$(CH_2)_2$-S-$R^2$    VIII

wherein $R^2$ is as defined above, is reacted with a compound of formula V, wherein R' is as defined above, and a compound of formula VII. wherein R is defined as above, to give a compound of formula I; or

e) A compound of formula VIII, wherein $R^2$ is defined as above, is reacted with a compound of formula VI, wherein R' is as defined above, and a compound of formula VII, wherein R is as defined above, in the presence of ammonia to give a compound of formula I.

**4.** Use of 4-alkyl-1-1,4-dihydropyridines of formula (I):

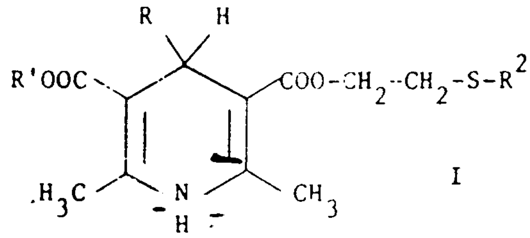

I

wherein,

R is a $C_1$-$C_4$ saturated straight or branched chain alkyl group,

R' may be a $C_1$-$C_6$ saturated straight or branched chain alkyl group, which may be interrupted by an oxygen atom. It may also represent the 2-tetrahydrofurfuryl group,

$R^2$ may be a $C_1$-$C_4$ saturated straight chain alkyl group or may represent a phenyl group,

as a PAF-antagonist.

**5.** Use, according to claim 4, of the compounds of formula (I) for the treatment of pathological states and diseases in which PAF participates.

**6.** Use, according to claims 4 and 5, of the compounds of formula (I) for the treatment of inflammatory processes of thetracheobronchial tree, acute and chronic bronchitis, bronchial asthma, states of shock and/or allergies.

**7.** Use, according to claims 4 to 6, of α-(phenylthio)ethyl-2,6-dimethyl-5-ethoxycarbonyl-4-methyl-1,4-dihydropyridin-3-carboxylate.

**Claims for the following Contracting States : ES, GR**

**1.** Process for the preparation of 4-alkyl-1,4-dihydropyridines of formula (I):

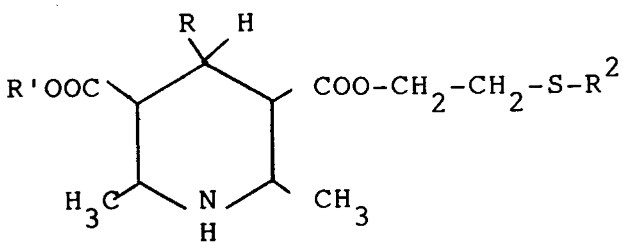

I

wherein,

R is a $C_1$-$C_4$ saturated straight or branched chain alkyl group,

R' may be a $C_1$-$C_6$ saturated straight or branched chain alkyl group, which may be interrupted by an oxygen atom. It may also represent the 2-tetrahydrofurfuryl group,

$R^2$ may be a $C_1$- $C_4$ saturated straight chain alkyl group or may represent a phenyl group,

with the proviso that the compound wherein R is methyl, R' is ethyl and $R^2$ is phenyl is excluded from said formula I

characterized because:

a) A compound of formula II

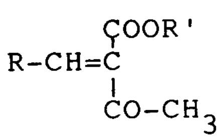

II

wherein R and R' are as defined above, is reacted with a compound of formula III

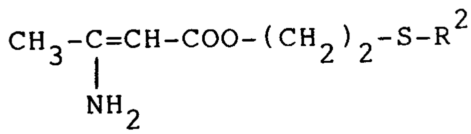

III

wherein $R^2$ is as defined above to give a compound of formula I; or

b) A compound of formula IV

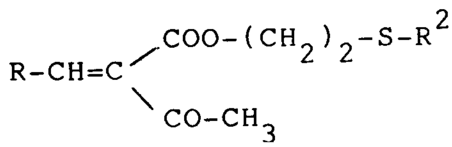

IV

wherein R and $R^2$ are defined as above, is reacted with a compound of formula V

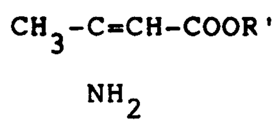

V

wherein $R'$ is as defined above, to give a compound of formula I; or

c) A compound of formula VI

$CH_3$-CO-$CH_2$COOR′ VI

wherein $R'$ is as defined above, is reacted with a compound of formula III, wherein $R^2$ is as defined above, and a compound of formula VII

R-CHO VII

wherein R is as defined above, to give a compound of formula I; or

d) A compound of formula VIII

$CH_3$-CO-$CH_2$-COO-$(CH_2)_2$-S-$R^2$ VIII

wherein $R^2$ is as defined above, is reacted with a compound of formula V wherein $R'$ is as defined above, and a compound of formula VII, wherein R is as defined above, to give a compound of formula I; or

e) A compound of formula VIII, wherein $R^2$ is as defined about, is reacted with a compound of formula VI, wherein $R'$ is as defined above, and a compound of formula VII, in which R is as defined above, in the presence of ammonia to give a compound of formula I

**2.** Process according to claim 1 wherein the compound (I) obtained is selected from among the following:

a) 2-(ethylthio)ethyl-2,6-dimethyl-4-methyl-5-methoxycarbonyl-1,4-dihydropyridin-3-carboxylate,
b) 2-(methylthio)ethyl-2,6-dimethyl-4-methyl-5-methoxycarbonyl-1,4-dihydropyridin-3-carboxylate,
c) 2-(methylthio)ethyl-2,6-dimethyl-5-ethoxycarbonyl-4-methyl-1,4-dihydropyridin-3-carboxylate,
d) 2-(ethylthio)ethyl-2,6-dimethyl-5-ethoxycarbonyl-4-methyl-1,4-dihydropyridin-3-carboxylate,
e) 2-(methylthio)ethyl-2,6-dimethyl-4-methyl-5-(2-methoxyethoxycarbonyl)-1,4-dihydropyrdind-3-carboxylate,
f) 2-(methylthio)ethyl-2,6-dimethyl-4-ethyl-5-ethoxycarbonyl-1,4-dihydropyridin-3-carboxylate,
g) 2-(methylthio)ethyl-2,6-dimethyl-5-ethoxycarbonyl-4-n-propyl-1,4-dihydropyridin-3-carboxylate,
h) 2-(ethylthio)ethyl-2,6-dimethyl-5-ethoxycarbonyl-4-n-propyl-1,4-dihydropyridin-3-carboxylate,
i) 2-(phenylthio)ethyl-2,6-dimethyl-4-methyl-5-(2-tetrahydrofurfuryloxycarbonyl)-1,4-dihydropyridin-3-carboxylate,
j) 2-(phenylthio)ethyl-2,6-dimethyl-5-isopropoxycarbonyl-4-methyl-1,4-dihydropyridin-3-carboxylate

k) 2 -(phenylthio)ethyl-2,6-dimethyl-4-methyl-5-methoxycarbonyl-1,4-dihydropyridin-3-carboxylate

**Patentansprüche**
**Patentansprüche für folgende Vertragsstaaten : AT, BE, CH, FR, GB, IT, LI, LU, NL, SE**

**1.** 4-Alkyl-1,4-dihydropyridine mit einer Aktivität als PAF-Antagonisten, der Formel I

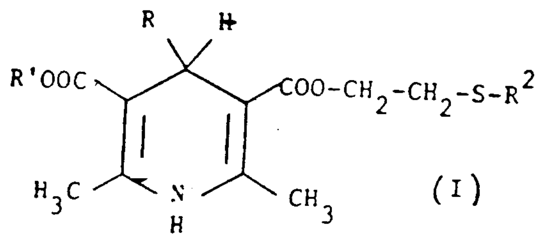

(I)

worin :

R eine gesättigte Alkylgruppe mit einer unverzweigten oder verzweigten $C_1$-$C_4$-Kette ist,
R' eine gesättigte, unverzweigte oder verzweigte $C_1$-$C_6$-Alkylgruppe, die durch ein Sauerstoffatom unterbrochen sein kann, Es kann auch eine 2-Tetrahydrofurfuryl-Gruppe darstellen,
$R^2$ eine gesättigte unverzweigte $C_1$-$C_4$-Alkylgruppe sein kann oder eine Phenylgruppe darstellt mit der Maßgabe, daß aus der Formel (I)
die Verbindung ausgeschlossen ist, in der R = Methyl, R' = Ethyl und $R^2$ = Phenyl ist.

**2.** Verbindung nach Anspruch 1, ausgewählt unter den folgenden:
(a) 2-(Ethylthio)ethyl-2,6-dimethyl-4-methyl-5-methoxycarbonyl-1,4-dihydropyridin-3-carboxylat,
(b) 2-(Methylthio)ethyl-2,6-dimethyl-4-methyl-5-methoxycarbonyl-1,4-dihydropyridin-3-carboxylat,
(c) 2-(Methylthio)ethyl-2,6-dimethyl-5-ethoxycarbonyl-4-methyl-1,4-dihydropyridin-3-carboxylat,
(d) 2-(Ethylthio)ethyl-2,6-dimethyl-5-ethoxycarbonyl-4-methyl-1,4-dihydropyridin-3-carboxylat,
(e) 2-(Methylthio)ethyl-2,6-dimethyl-4-methyl-5-(2-methoxyethoxycarbonyl)-1,4-dihydropyridin-3-carboxylat,
(f) 2-(Methylthio)ethyl-2,6-dimethyl-4-ethyl-5-ethoxycarbonyl-1,4-dihydropyridin-3-carboxylat,
(g) 2-(Methylthio)ethyl-2,6-dimethyl-5-ethoxycarbonyl-4-n-propyl-1,4-dihydropyridin-3-carboxylat,
(h) 2-(Ethylthio)ethyl-2,6-dimethyl-5-ethoxycarbonyl-4-n-propyl-1,4-dihydropyridin-3-carboxylat,
(i) 2-(Phenylthio)ethyl-2,6-dimethyl-4-methyl-5-(2-tetrahydrofurfuryloxycarbonyl)-1,4-dihydropyridin-3-carboxylat,
(j) 2-(Phenylthio)ethyl-2,6-dimethyl-5-isopropoxycarbonyl-4-methyl-1,4-dihydropyridin-3-carboxylat und
(k) 2-(Phenylthio)ethyl-2,6-dimethyl-4-methyl-5-methoxycarbonyl-1,4-dihydropyridin-3-carboxylat.

**3.** Verfahren zur Herstellung von 4-Alkyl-1,4-dihydropyridinen der Formel (I):

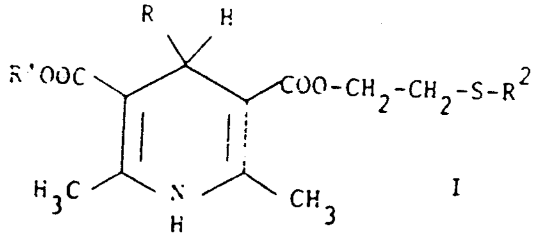

I

worin :

R eine gesättigte, unverzweigte oder verzweigte $C_1$-$C_4$-Alkylgruppe ist,
R' eine gesättigte unverzweigte oder verzweigte $C_1$-$C_6$-Alkylgruppe sein kann, die durch ein

Sauerstoffatom unterbrochen sein kann. Es kann auch die 2-Tetrahydrofurfurylgruppe bedeuten,

$R^2$ eine unverzweigte gesättigte $C_1$-$C_4$-Alkylgruppe oder eine Phenylgruppe, sein kann, mit der Massgabe, dass die Verbindung in der R = Methyl, R' = Äthyl und $R^2$ = Phenyl von besagter Formel I ausgeschossen ist

dadurch gekennzeichnet, dass man

(a) eine Verbindung der allgemeinen Formel

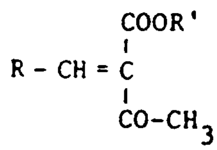

(II)

worin R und R' wie oben definiert sind, mit einer Verbindung der Formel III umsetzt

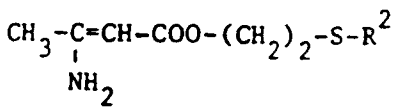

III

worin $R^2$ wie oben definiert ist,
zur Bildung einer Verbindung der Formel (I);
oder
(b) eine Verbindung der Formel IV

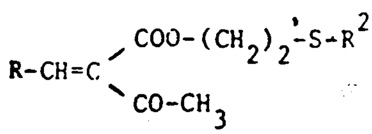

IV

worin R und $R^2$ wie oben definiert sind, mit einer Verbindung der Formel V umsetzt

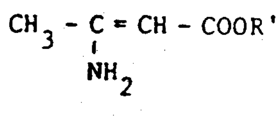

V

worin R' wie oben definiert ist,
zur Bildung einer Verbindung der Formel I;
oder
(c) eine Verbindung der Formel VI

$CH_3$-CO-$CH_2$-COOR' VI

worin R' wie oben definiert ist,
mit einer Verbindung der Formel III, worin $R^2$ wie oben definiert ist, und einer Verbindung der Formel VII umsetzt

R - CHO VII

worin R wie oben definiert ist, zur Bildung einer Verbindung der Formel I; oder
(d)eine Verbindung der Formel VIII

$CH_3$-CO-$CH_2$-COO-$(CH_2)_2$-S-$R^2$ VIII

worin $R^2$ wie oben definiert ist, mit einer Verbindung der Formel V , worin R' wie oben definiert ist, und einer Verbindung der Formel VIII, worin R wie oben definiert ist, umsetzt zur Bildung einer Verbindung der Formel I; oder

(e) eine Verbindung der Formel VIII, worin $R^2$ wie oben definiert ist, mit einer Verbindung der Formel VI , worin R' wie oben definiert ist, und einer Verbindung der Formel VII , worin R wie oben definiert ist, in Gegenwart von Ammoniak umsetzt zur Bildung einer Verbindung der Formel I.

**4.** Verwendung der 4-Alkyl-1,4-dihydropyridine der Formel

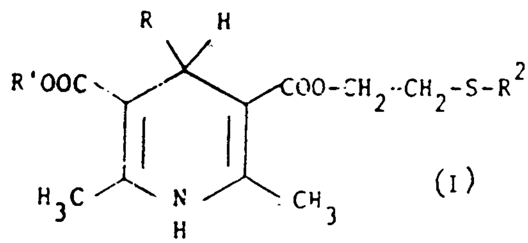

(I)

worin bedeuten:

R eine gesättigte, unverzweigte oder verzweigte $C_1$-$C_4$-Alkylgruppe,

R' eine gesättigte unverzweigte oder verzweigte $C_1$-$C_6$-Alkylgruppe, die durch ein Sauerstoffatom unterbrochen sein kann. Es kann auch die 2-Tetrahydrofurfurylgruppe darstellen

$R^2$ eine gesättigte unverzweigte $C_1$-$C_4$-Alkylgruppe oder eine Phenylgruppe sein kann,

als PAF-Antagonisten.

**5.** Verwendung nach Anspruch 4 der Verbindungen der Formel (I) für die Behandlung von pathologischen und krankhaften Zuständen, an denen PAF beteiligt ist.

**6.** Verwendung nach Anspruch 4 und 5 der Verbindungen der Formel (I) für die Behandlung von inflamatorischen Prozessen der Luftröhre, der akuten und chronischen Bronchitis, des Asthma bronchiale, von Schock- und/oder Allergie-Zuständen.

**7.** Verwendung nach den Ansprüchen 4 bis 6 von $\alpha$-(Phenylthio)ethyl-2,6-dimethyl-5-ethoxycarbonyl-4-methyl-1,4-dihydropyridin-3-carboxylat.

## Patentansprüche für folgende Vertragsstaaten : ES, GR

**1.** Verfahren zur Herstellung von 4-Alkyl-1,4-dihydropyridinen der Formel (I)

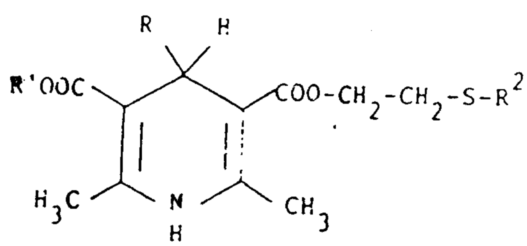

worin

R eine gesättigte, unverzweigte oder verzweigte $C_1$-$C_4$-Alkylgruppe ist,

R' eine gesättigte unverzweigte oder verzweigte $C_1$-$C_6$-Alkylgruppe die durch ein Sauerstoffatom unterbrochen sein kann. Es kann auch die 2-Tetrahydrofurfurylgruppe darstellen

$R^2$ eine unverzweigte gesättigte $C_1$-$C_4$ -Alkylgruppe oder eine Phenylgruppe, unter der Massgabe, dass die Verbindung in der R = Methyl, R' Äthyl und $R^2$ = Phenyl ist, von besagter Formel I ausgeschlossen ist,

dadurch gekennzeichnet, daß man

(a) eine Verbindung der Formel II

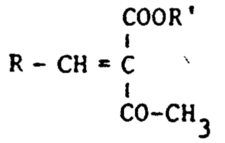
(II)

worin R und R' wie oben definiert sind, mit einer Verbindung der Formel (III) umsetzt

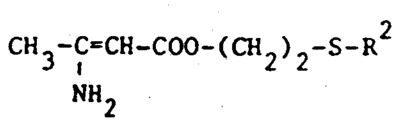
(III)

worin $R^2$ wie oben definiert ist,
zur Bildung einer Verbindung der Formel (I);
oder

(b) eine Verbindung der Formel IV

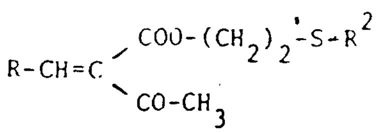
(IV)

worin R und $R^2$ wie oben definiert sind, mit einer Verbindung der Formel (V) umsetzt

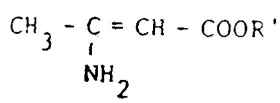
(V)

worin R' wie oben definiert ist,
zur Bildung einer Verbindung der
oder

(c) eine Verbindung der Formel VI

$CH_3$-CO-$CH_2$-COOR' (VI)

worin R' wie oben definiert ist;
mit einer Verbindung der Formel (III), worin $R^2$
wie oben definiert ist, und einer Verbindung der allgemeinen Formel (VII) umsetzt

R - CHO (VII)

worin R wie oben definiert ist, zur Bildung einer Verbindung der Formel (I); oder

(d)eine Verbindung der allgemeinen Formel VIII

$CH_3$-CO-$CH_2$-COO-$(CH_2)_2$-S-$R^2$ (VIII)

worin $R^2$ wie oben definiert ist, mit einer Verbindung der Formel (V), worin R' wie oben definiert ist, und einer Verbindung der Formel (VII), worin R wie oben definiert ist, umsetzt zur Bildung einer Verbindung der Formel (I).

(e) eine Verbindung der Formel (VIII), worin $R^2$ wie oben definiert ist, mit einer Verbindung der Formel (VI), worin R' wie oben definiert ist, und einer Verbindung der Formel (VII), worin R wie oben definiert ist, in Gegenwart von Ammoniak umsetzt zur Bildung einer Verbindung der allgemeinen Formel (I).

**2.** Verfahren nach Anspruch 1, bei dem die erhaltene Verbindung (I) unter den folgenden ausgewählt wird:

(a) 2-(Ethylthio)ethyl-2,6-dimethyl-4-methyl-5-methoxycarbonyl-1,4-dihydropyridin-3-carboxylat,
(b) 2-(Methylthio)ethyl-2,6-dimethyl-4-methyl-5-methoxycarbonyl-1,4-dihydropyridin-3-carboxylat,
(c) 2-(Methylthio)ethyl-2,6-dimethyl-5-ethoxycarbonyl-4-methyl-1,4-dihydropyridin-3-carboxylat,
(d) 2-(Ethylthio)ethyl-2,6-dimethyl-5-ethoxycarbonyl-4-methyl-1,4-dihydropyridin-3-carboxylat,
(e) 2-(Methylthio)ethyl-2,6-dimethyl-4-methyl-5-(2-methoxyethoxycarbonyl)-1,4-dihydropyridin-3-carboxylat,
(f) 2-(Methylthio)ethyl-2,6-dimethyl-4-ethyl-5-ethoxycarbonyl-1,4-dihydropyridin-3-carboxylat,
(g) 2-(Methylthio)ethyl-2,6-dimethyl-5-ethoxycarbonyl-4-n-propyl-1,4-dihydropyridin-3-carboxylat,
(h) 2-(Ethylthio)ethyl-2,6-dimethyl-5-ethoxycarbonyl-4-n-propyl-1,4-dihydropyridin-3-carboxylat,
(i) 2-(Phenylthio)ethyl-2,6-dimethyl-4-methyl-5-(2-tetrahydrofurfuryloxycarbonyl)-1,4-dihydropyridin-3-carboxylat,
(j) 2-(Phenylthio)ethyl-2,6-dimethyl-5-isopropoxycarbonyl-4-methyl-1,4-dihydropyridin-3-carboxylat und
(k) 2-(Phenylthio)ethyl-2,6,dimethyl-4-methyl-5-methoxycarbonyl-1,4-dihydropyridin-3-carboxylat

## Revendications

**Revendications pour les Etats contractants suivants : AT, BE, CH, FR, GB, IT, LI, LU, NL, SE**

**1.** 4-alkyl-1,4-dihydropyridines douées d'activité antagoniste de PAF, de formule I

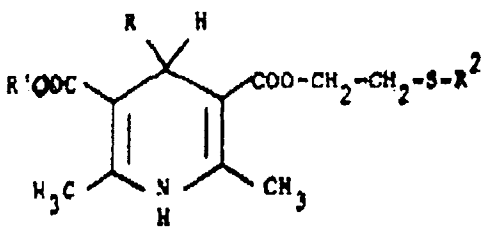

I

où

R est un groupe alkyle saturé en $C_1$-$C_4$ à chaîne droite ou ramifiée,

R' peut être un groupe alkyle saturé en $C_1$-$C_6$ à chaîne droite ou ramifiée, qui peut être interrompu par un atome d'oxygène ; il peut également représenter un groupe 2-tétrahydrofurfuryle,

$R^2$ peut être un groupe alkyle saturé en $C_1$-$C_4$ à chaîne droite ou peut représenter un groupe phényle,

à condition que le composé dans lequel R est le groupe méthyle, R' est le groupe éthyle et $R^2$ est le groupe phényle soit exclu de ladite formule I.

**2.** Composé selon la revendication 1, choisi parmi les suivants :

a) 2,6-diméthyl-4-méthyl-5-méthoxycarbonyl-1,4-dihydropyridine-3-carboxylate de 2-(éthylthio)éthyle,
b) 2,6-diméthyl-4-méthyl-5-méthoxycarbonyl-1,4-dihydropyridine-3-carboxylate de 2-(méthylthio)-éthyle,
c) 2,6-diméthyl-5-éthoxycarbonyl-4-méthyl-1,4-dihydropyridine-3-carboxylate de 2-(méthylthio)éthyle,
d) 2,6-diméthyl-5-éthoxycarbonyl-4-méthyl-1,4-dihydropyridine-3-carboxylate de 2-(éthylthio)éthyle,
e) 2,6-diméthyl-4-méthyl-5-(2-méthoxyéthoxycarbonyl)-1,4-dihydropyridine-3-carboxylate de 2-(méthylthio)éthyle,
f) 2,6-diméthyl-4-éthyl-5-éthoxycarbonyl-1,4-dihydropyridine-3-carboxylate de 2-(méthylthio)éthyle,

g) 2,6-diméthyl-5-éthoxycarbonyl-4-*n*-propyl-1,4-dihydropyridine-3-carboxylate de 2-(méthylthio)-éthyle,

h) 2,6-diméthyl-5-éthoxycarbonyl-4-*n*-propyl-1,4-dihydropyridine-3-carboxylate de 2-(éthylthio)éthyle,

i) 2,6-diméthyl-4-méthyl-5-(2-tétrahydrofurfuryloxycarbonyl)-1,4-dihydropyridine-3-carboxylate de 2-(phénylthio)-éthyle,

j) 2,6-diméthyl-5-isopropoxycarbonyl-4-méthyl-1,4-dihydropyridine-3-carboxylate de 2-(phénylthio)-éthyle,

k) 2,6-diméthyl-4-méthyl-5-méthoxycarbonyl-1,4-dihydropyridine-3-carboxylate de 2,6-(phénylthio)-éthyle.

**3.** Procédé pour la préparation de 4-alkyl-1,4-dihydropyridines de formule (I) :

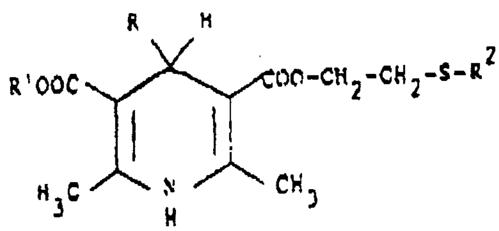

I

où

R est un groupe alkyle saturé en $C_1$-$C_4$ à chaîne droite ou ramifiée,

R' peut être un groupe alkyle saturé en $C_1$-$C_6$ à chaîne droite ou ramifiée, qui peut être interrompu par un atome d'oxygène ; il peut également représenter un groupe 2-tétrahydro-furfuryle,

$R^2$ peut être un groupe alkyle saturé en $C_1$-$C_4$ à chaîne droite ou peut représenter un groupe phényle,

à condition que le composé' dans lequel R est le groupe méthyle, R' est le groupe éthyle et $R^2$ est le groupe phényle soit exclu de ladite formule I,

caractérisé en ce que :

a) un composé de formule II

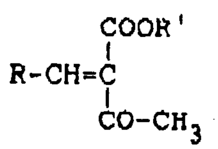

II

où R et R' sont tels que définis ci-dessus, est amené à réagir avec un composé de formule III

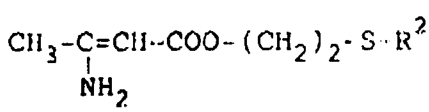

III

où $R^2$ est tel que défini ci-dessus, pour donner un composé de formule I ; ou

b) un composé de formule IV

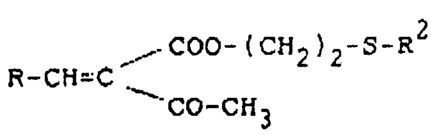

IV

où R et $R^2$ sont tels que défini ci-dessus, est amené à réagir avec un composé de formule V

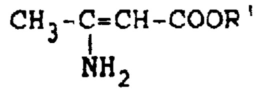

V

où R' est tel que défini ci-dessus, pour donner un composé de formule I ; ou
c) un composé de formule VI

$CH_3$-CO-$CH_2$-COOR'  VI

où R' est tel que défini ci-dessus, est amené à réagir avec un composé de formule III, où $R^2$ est tel que défini ci-dessus, et un composé de formule VII

R-CHO  VII

où R est tel que défini ci-dessus, pour donner un composé de formule I ; ou
d) un composé de formule VIII

$CH_3$-CO-$CH_2$-COO-$(CH_2)_2$-S-$R^2$  VIII

où $R^2$ est tel que défini ci-dessus, est amené à réagir avec un composé de formule V, où R' est tel que défini ci-dessus, et un composé de formule VII, où R est tel que défini ci-dessus, pour donner un composé de formule I ; ou
e) un composé de formule VIII, où $R^2$ est tel que défini ci-dessus, est amené à réagir avec un composé de formule VI, où R' est tel que défini ci-dessus, et un composé de formule VII, où R est tel que défini ci-dessus, en présence d'ammoniac, pour donner un composé de formule I.

**4.** Utilisation de 4-alkyl-1,4-dihydropyridines de formule (I) :

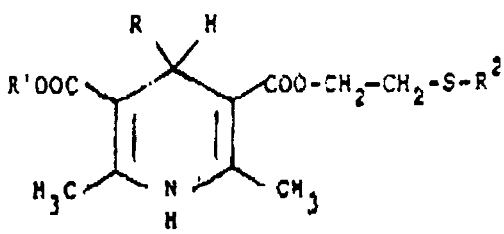

I

où

- R  est un groupe alkyle saturé en $C_1$-$C_4$ à chaîne droite ou ramifiée,
- R'  peut être un groupe alkyle saturé en $C_1$-$C_6$ à chaîne droite ou ramifiée, qui peut être interrompu par un atome d'oxygène ; il peut également représenter un groupe 2-tétrahydrofurfuryle,
- $R^2$  peut être un groupe alkyle saturé en $C_1$-$C_4$ à chaîne droite ou peut représenter un groupe phényle,

comme antagonistes de PAF.

**5.** Utilisation selon la revendication 4 des composés de formule (I) pour le traitement d'états pathologiques et d'affections auxquels participe PAF.

**6.** Utilisation selon les revendications 4 et 5 des composés de formule (I) pour traiter des processus inflammatoires de l'arbre trachéo-bronchique, la bronchite aiguë et chronique, l'asthme bronchique, des états de choc et/ou des allergies.

**7.** Utilisation selon les revendications 4 à 6 du 2,6-diméthyl-5-éthoxycarbonyl-4-méthyl-1,4-dihydropyridine-3-carboxylate de $\alpha$-(phénylthio)éthyle.

**Revendications pour les Etats contractants suivants : ES, GR**

**1.** Procédé pour la préparation de 4-alkyl-1,4-dihydropyridines de formule (I) :

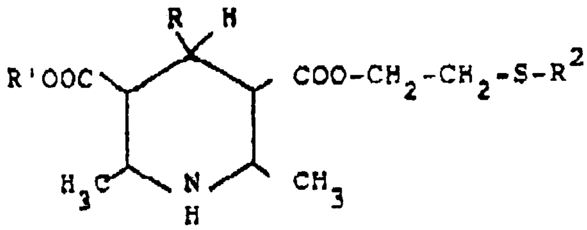

I

où

R est un groupe alkyle saturé en $C_1$-$C_4$ à chaîne droite ou ramifiée,

R' peut être un groupe alkyle saturé en $C_1$-$C_6$ à chaîne droite ou ramifiée, qui peut être interrompu par un atome d'oxygène ; il peut également représenter un groupe 2-tétrahydrofurfuryle,

$R^2$ peut être un groupe alkyle saturé en $C_1$-$C_4$ à chaîne droite ou peut représenter un groupe phényle,

à condition que le composé dans lequel R est le groupe méthyle, R' est le groupe éthyle et $R^2$ est le groupe phényle soit exclu de ladite formule I,

caractérisé en ce que :

a) un composé de formule II

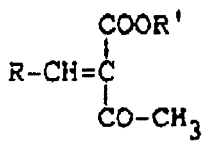

II

où R et R' sont tels que définis ci-dessus, est amené à réagir avec un composé de formule III

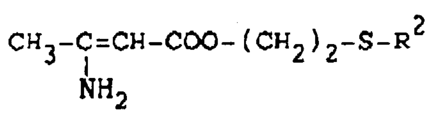

III

où $R^2$ est tel que défini ci-dessus, pour donner un composé de formule I ; ou

b) un composé de formule IV

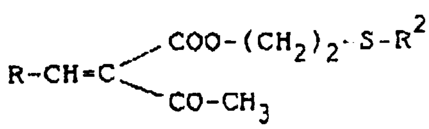

IV

où R et $R^2$ sont tels que défini ci-dessus, est amené à réagir avec un composé de formule V

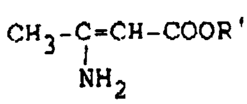

V

où R' est tel que défini ci-dessus, pour donner un composé de formule I : ou

c) un composé de formule VI

$CH_3$-CO-$CH_2$-COOR'      VI

où R' est tel que défini ci-dessus, est amené à réagir avec un composé de formule III, où $R^2$ est tel que défini ci-dessus, et un composé de formule VII

R-CHO      VII

où R est tel que défini ci-dessus, pour donner un composé de formule I ; ou
d) un composé de formule VIII

$CH_3$-CO-$CH_2$-COO-$(CH_2)_2$-S-$R^2$      VIII

où $R^2$ est tel que défini ci-dessus, est amené à réagir avec un composé de formule V, où R' est tel que défini ci-dessus, et un composé de formule VII, où R est tel que défini ci-dessus, pour donner un composé de formule I ; ou
e) un composé de formule VIII, où $R^2$ est tel que défini ci-dessus, est amené à réagir avec un composé de formule VI, où R' est tel que défini ci-dessus, et un composé de formule VII, où R est tel que défini ci-dessus, en présence d'ammoniac, pour donner un composé de formule I.

**2.** Procédé selon la revendication 1, dans lequel le composé (I) obtenu est choisi parmi les suivants :
a) 2,6-diméthyl-4-méthyl-5-méthoxycarbonyl-1,4-dihydropyridine-3-carboxylate de 2-(éthylthio)éthyle,
b) 2,6-diméthyl-4-méthyl-5-méthoxycarbonyl-1,4-dihydropyridine-3-carboxylate de 2-(méthylthio)-éthyle,
c) 2,6-diméthyl-5-éthoxycarbonyl-4-méthyl-1,4-dihydropyridine-3-carboxylate de 2-(méthylthio)éthyle,
d) 2,6-diméthyl-5-éthoxycarbonyl-4-méthyl-1,4-dihydropyridine-3-carboxylate de 2-(éthylthio)éthyle,
e) 2,6-diméthyl-4-méthyl-5-(2-méthoxyéthoxycarbonyl)-1,4-dihydropyridine-3-carboxylate de 2-(méthylthio)éthyle,
f) 2,6-diméthyl-4-éthyl-5-éthoxycarbonyl-1,4-dihydropyridine-3-carboxylate de 2-(méthylthio)éthyle,
g) 2,6-diméthyl-5-éthoxycarbonyl-4-*n*-propyl-1,4-dihydropyridine-3-carboxylate de 2-(méthylthio)-éthyle,
h) 2,6-diméthyl-5-éthoxycarbonyl-4-*n*-propyl-1,4-dihydropyridine-3-carboxylate de 2-(éthylthio)éthyle,
i) 2,6-diméthyl-4-méthyl-5-(2-tétrahydrofurfuryloxycarbonyl)-1,4-dihydropyridine-3-carboxylate de 2-(phénylthio)-éthyle,
j) 2,6-diméthyl-5-isopropoxycarbonyl-4-méthyl-1,4-dihydropyridine-3-carboxylate de 2-(phénylthio)-éthyle,
k) 2,6-diméthyl-4-méthyl-5-méthoxycarbonyl-1,4-dihydropyridine-3-carboxylate de 2,6-(phénylthio)-éthyle.